# EUROPÄISCHE PATENTSCHRIFT

(11) **EP 0 922 647 B1**
(45) Veröffentlichungstag und Bekanntmachung des Hinweises auf die Patenterteilung: **18.07.2001**
(21) Anmeldenummer: 98121450.5
(22) Anmeldetag: 11.11.1998
(51) Int. Cl.: B65D 51/00, A61J 1/00, A61M 5/315, B05D 7/24, C23C 16/54, C23C 16/40

(54) **Verfahren zum Beschichten von Elastomerkomponenten**
Process for coating elastomer components
Procédé de revêtement d'éléments élastomériques

(30) Priorität: 05.12.1997 DE 19754056
(43) Veröffentlichungstag der Anmeldung: 16.06.1999
(73) Patentinhaber: Schott Glas, 55122 Mainz (DE); CARL-ZEISS-STIFTUNG trading as SCHOTT GLAS, 55122 Mainz (DE)
(72) Erfinder: Spallek, Michael, Dr., 55218 Ingelheim (DE); Walther, Marten, Dr., 55270 Engelstadt (DE); Danielzik, Burkhard, Dr., 55218 Ingelheim (DE); Kuhr, Markus, Dr., 55579 Wöllstein (DE)
(74) Vertreter: Fuchs Mehler Weiss & Fritzsche

(56) Entgegenhaltungen:
- EP-A- 0 148 426
- DE-A- 4 438 359
- US-A- 5 064 083

## Beschreibung

Die Erfindung bezieht sich auf ein Verfahren zum Beschichten von Elastomerkomponenten für medizinische Zwecke.

Elastomerkomponenten für Anwendungen, bei denen es auf pharmaverträgliche Aspekte des Elastomermaterials ankommt, sind in vielen Erscheinungsformen bekannt, insbesondere als Verschlüsse von Behältern für Lebensmittel und Pharmaverpackungen, wie Injektionsstopfen, Infusionsstopfen oder Kolbenstopfen für Spritzenkörper. So verlangt z.B. die DIN ISO 11040, Teil 2 von den aus einem Elastomermaterial bestehenden Stopfen für eine Dentalkarpule, daß er keine Substanz abgeben darf, welche die therapeutische Wirkung der Injektionspräparate ungünstig beeinflussen könnte.

Die Anforderungen für Gummiteile, d.h. Teile, die aus einem elastomeren Werkstoff gefertigt werden, im medizinischen Bereich, die während der Herstellung, Lagerung und Anwendung von wässrigen Lösungen-Suspensionen - mit diesen in Berührung kommen, werden generell durch die Norm DIN 58 367, Teil 1 festgelegt.

Ähnlich liegen die Randbedingungen für Elastomerkomponenten bei vergleichbaren Anwendungen.

Es ist bekannt, durch die Wahl eines geeigneten Elastomermaterials die vorgenannten Bedingungen soweit wie möglich zu erfüllen. Dabei muß man jedoch Kompromisse mit anderen, insbesondere mechanischen Eigenschaften eingehen, was in vielen Fällen als nachteilig empfunden wird.

Es ist auch bekannt, die Elastomerkomponenten mit einer äußeren Beschichtung zu versehen, die die notwendige Inertheit schafft, so daß die anderen notwendigen Eigenschaften der beschichteten Elastomerkomponenten freier gewählt werden können.

Im bekannten Fall wird jede Elastomerkomponente für sich beschichtet.

So beschreibt die EP 0 497 567 B1 einen Sanitärartikel aus Gummi, insbesondere einen Stopfen, dessen Oberfläche mit einem Kunststoffilm laminiert ist, der aus einem cyklischen Olefincopolymer (COC) besteht.

Die EP 0 296 878 B1 beschreibt einen Sanitär-Kautschukgegenstand, dessen Oberfläche mit einer durch Strahlen vernetzten Schicht aus einem modifizierten Polysiloxan versehen ist, wobei diese Schicht aus einer flüssigen Phase heraus aufgebracht wird.

Es ist fertigungstechnisch relativ aufwendig, die einzelnen Elastomerkomponenten zu beschichten. Daher beschreibt die WO 96/349 26 ein Plasmabeschichtungs-Verfahren zur summarischen Beschichtung von Elastomerkomponenten, die sich in einer Beschichtungstrommel, in der die Elastomerkomponenten bewegt werden, befinden. Dieses Verfahren gewährleistet jedoch keine definiert gleichförmige vollständige Beschichtung der einzelnen Elastomerkomponenten, da Abschattungs- oder Abriebeffekte auftreten. Damit muß für eine "Mindestdicke" der Beschichtung auf allen Teilen im Mittel eine erhebliche Überdimensionierung der Schicht vorgenommen werden.

Ein weiteres Verfahren zur summarischen Beschichtung von Elastomerkomponenten ist durch die US-A-5,064,083 bekannt geworden. Bei diesem Verfahren werden die Elastomerkomponenten einzeln in rasterartig ausgeformte Aushöhlungen einer Platte gebracht und in bestimmten Abschnitten mit Polyparaxylen beschichtet. Über das Beschichtungsverfahren selbst finden sich in dieser Schrift keine konkreten Angaben.

Auch dieses Verfahren ist durch die Einzelbehandlung der Elastomerkomponenten, die zudem in den Aushöhlungen genau ausgerichtet sein müssen, damit nur die gewünschten Abschnitte beschichtet werden, sehr aufwendig.

Aufgabe der Erfindung ist die Entwicklung eines Verfahrens zur Beschichtung der eingangs bezeichneten Elastomerkomponenten, welches eine Beschichtung aus der Gasphase in einem kontinuierlichen Prozeß ermöglicht, wobei die Elastomerkomponenten nur teilweise, zumindest in den Abschnitten, in denen sie mit der pharmazeutischen Lösung in Berührung kommen, beschichtet werden und keine Abschattungs- oder Abriefeffekte auftreten. Die Beschichtung soll dabei die Inertheit und das Gleit- und Haftreibungsverhalten der Elastomerkomponenten verbessern.

Die Lösung dieser Aufgabe gelingt ausgehend von dem eingangs bezeichneten Verfahren mit den Schritten:
- Herstellen einer ausgedehnt flächigen Matte aus dem Elastomer-Werkstoff mit einer Vielzahl von zusammenhängend angeformten vorstehenden Elastomerkomponenten,
- Beschichten mindestens der Seite der Matte mit den vorstehenden Elastomerkomponenten mittels eines "kalten" Beschichtungsverfahrens, typischerweise nach dem plasmaunterstützten PECVD-Verfahren (PECVD-Verfahren), in einem kontinuierlichen Prozeß mit einer Schicht, die eine Inertheit der Elastomerkomponenten und ihr Gleit- sowie Haftreibungsverhalten verbessert, und
- Ausstanzen der Elastomerkomponenten aus der Matte.

Mit Hilfe des erfindungsgemäßen Verfahrens können die Elastomerkomponenten auf kostengünstige und dennoch sehr wirksame Weise mit der gewünschten Beschichtung versehen werden, wobei diese Schicht nur an den Stellen aufgebracht wird, an denen die Elastomerkomponenten mit der medizinisch bzw. pharmazeutisch wirksamen Lösung in Kontakt tritt. Dadurch kann die Menge des zugeführten Beschichtungsgases sowie die Produktionszeit maßgebend gesenkt werden, was sich mit Vorteil günstig auf die Herstellungskosten auswirkt.

Gemäß einer Weiterbildung der Erfindung besteht die Schicht vorzugsweise aus einer Siliziumdioxid-Schicht, deren Eigenschaften durch den Einbau von Kohlenstoff, Wasserstoff und Stickstoff von harten Schichten bis hin zu polymerartigen Eigenschaften beeinflußt werden kann. Eine derartige Schicht gewährleistet die notwendige Inertheit als auch das gewünschte Gleitverhalten der Elastomerkomponenten. Zusätzlich können andere Schichtmaterialien in diese Grundschicht eingebettet werden, die eine weitere Steigerung der Inertheit bewirken können.

Zu diesem Zweck wird gemäß einer Ausgestaltung der Erfindung als Beschichtungsgas für das PECVD-Verfahren Tetramethyldisiloxan (TMDSO), Hexamethyldisiloxan (HMDSO) oder ähnliche Siloxane verwendet.

Alternativ können als Reaktionsgas für das PECVD-Verfahren Silazane verwendet werden.

Das PECVD-Verfahren (Plasma-Enhanced-Chemical-Vapor-Deposition) ist in unterschiedlichster Literatur beschrieben. Diverse Ausführungsformen werden mit unterschiedlichsten Energieeinspeisungen von Niederfrequenz (z.B. 40 kHz) über Mittelfrequenz (z.B. 13,56 MHz) bis zu Mikrowellen (2,45 GHz und darüber) eingesetzt. Als besonders vorteilhaft hat sich das Plasmaimpuls-CVD-Verfahren (PICVD) erwiesen, da eine hohe Homogenität bei der Beschichtung großflächiger Substrate erreicht werden kann. Beispiele dazu finden sich in G. Janzen: Plasmatechnik, Hütig Verlag, Heidelberg 1992).

Weitere ausgestaltende Merkmale der Erfindung ergeben sich anhand der Beschreibung eines in den Zeichnungen dargestellten Ausführungsbeispieles.

Es zeigen:
- Fig. 1: einen Ausschnitt aus einem Längsschnitt durch eine mit dem erfindungsgemäßen Verfahren beschichtete Matte aus Elastomerkomponenten,
- Fig. 2: eine bevorzugte Vorrichtung für das PICVD-Verfahren, mit dem typischerweise die Matten nach Fig. 1 beschichtet werden.

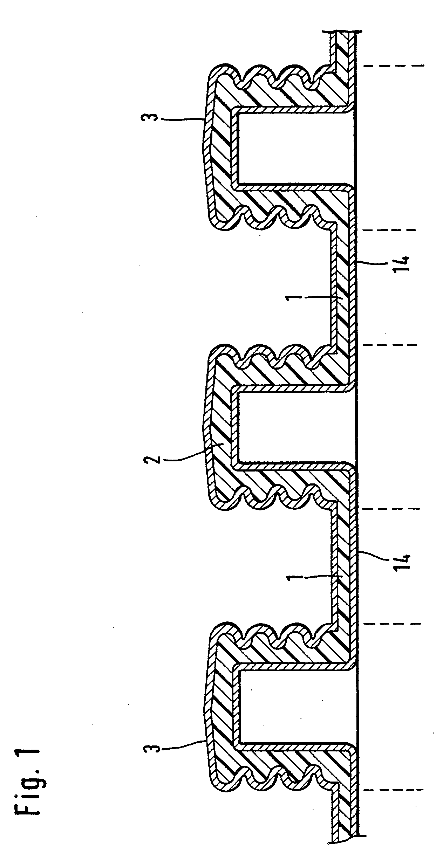

Die Fig. 1 zeigt einen Ausschnitt aus einem Längsschnitt durch eine ausgedehnt flächige Matte 1 aus dem gewünschten Elastomerwerkstoff mit einer Vielzahl von zusammenhängend angeformten Elastomerkomponenten 2. Diese Elastomerkomponenten sind in Fig. 1 schematisiert dargestellte Stopfen, wie sie z.B. in der EP 0 296 878, Fig. 1, gezeigt sind.

Diese Matten 1 mit den Elastomerkomponenten, die typischerweise eine Abmessung von 30 cm haben, werden in üblicher Weise mit bekannten Verfahren hergestellt, z.B. durch ein Preßverfahren oder auf dem Weg des Spritzgießens.

In einem nachfolgenden Prozeß wird die Seite der Matte 1 mit den nach oben vorstehenden Elastomerkomponenten in einem kontinuierlichen Prozeß nach dem PICVD-Verfahren beschichtet, und zwar mit einer oberen Schicht 3, die eine Inertheit der Elastomerkomponente und ihr Gleit- sowie Haftreibungsverhalten verbessert. Diese Schicht 3 ist vorzugsweise eine Siliziumdioxid-Schicht, deren Eigenschaften durch den Einbau von Kohlenstoff, Wasserstoff und Stickstoff von harten Schichten bis hin zu polymerartigen Eigenschaften beeinflußt werden kann.

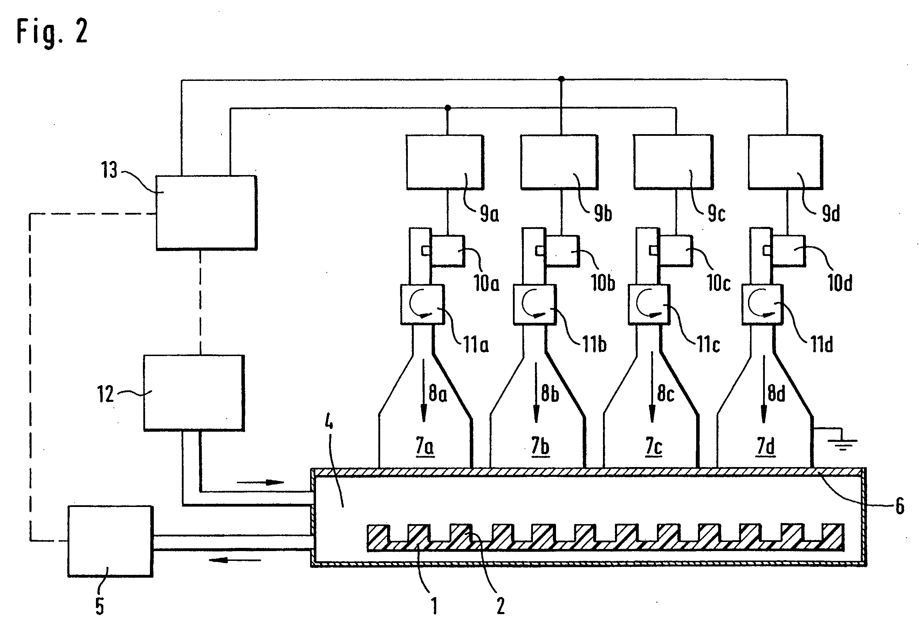

Nach dem Aufbringen der Schicht, was anhand der Fig. 2 noch näher erläutert werden wird, werden die Elastomerkomponenten 2 aus der Matte 1 ausgestanzt, wobei in Fig. 1 die Stanzlinien gestrichelt dargestellt sind.

Bei der Darstellung in Fig. 1 ist nur die Oberseite der Matte 1 beschichtet, d.h. die hervorstehenden Flächen der Elastomerkomponenten 2, die bei dem späteren Gebrauch mit der pharmazeutischen oder medizinischen Lösung in Kontakt treten. Die Oberseite des Stopfens entsprechend der Unterseite der Matte 1, d.h. das Stopfenäußere bei einer späteren Verwendung der Stopfen als Verschlußelement, ist dann (anders als dargestellt) unbeschichtet, so daß einmal das Beschichtungsmaterial eingespart wird und die Zeit für das Aufbringen der Schichten vermindert werden kann, da typischerweise nur die eine Seite der Matte beschichtet werden muß, nicht aber deren Unterseite.

Die Beschichtung nach dem PICVD-Verfahren gewährleistet zudem, daß keine Abschattungs- oder Abriebeffekte bei dem Beschichten der Elastomerkomponenten auftreten, sondern daß sich eine verhältnismäßig gleichmäßige Schicht 3 aufbaut. Ebenso ist die Beschichtung von Hinterschnitten gewährleistet.

Das Prinzip einer Durchlauf-Beschichtungsstation, in der das PICVD-Verfahren durchgeführt wird, ist in Fig. 2 dargestellt. In eine Vakuumkammer 4 wird fortlaufend die zu beschichtende Matte 1 mit den hervorstehenden Elastomerkomponenten 2 eingeführt. Diese Vakuumkammer 4, die als Reaktionskammer dient, kann mittels einer Vakuumeinrichtung 5, die typischerweise eine Vakuumpumpe und die notwendigen Armaturen aufweist, entlüftet werden, z.B. auf einen Druck von 0,3 mbar. Oberhalb der Vakuumkammer 4, getrennt durch ein Mikrowellenfenster 6, befindet sich eine Reihe von Horn-Mikrowellenantennen 7a bis 7d, die eine lineare Plasmaquelle bilden. Über diese Mikrowellenantennen wird eine Mikrowellenstrahlung 8a bis 8d impulsweise in die Vakuumkammer 4 geleitet, die im Innern der Vakuumkammer ein Mikrowellenplasma bilden. Die Impulsdauer ist dabei ein zusätzlicher Parameter, der die Zusammensetzung der abgeschiedenen Schicht beeinflußt.

Die Mikrowellenimpulse, deren Dauer im Bereich von 0,1 bis 10 ms liegt, werden von Mikrowellengeneratoren 9a bis 9d erzeugt, die jeweils über ein Magnetron 10a bis 10d und Einwegleitungen 11a bis 11d mit den Mikrowellenantennen 7a bis 7d verbunden sind. Die Mikrowellenanordnung besitzt typischerweise Standard-Komponenten der 2,45 GHz-Technologie.

Über eine Gaszufuhr-Anordnung 12 wird einmal das Gas, in welchem ein Plasmabogen gezündet wird, typischerweise Sauerstoff und zum anderen das zur Schichtbildung notwendige Gas, das Reaktionsgas, eingeleitet. Im vorliegenden Ausführungsbeispiel sind es typischerweise Siloxane, vorzugsweise Tetramethyldisiloxan, oder Hexamethyldisiloxan oder Silazane, aus denen über die Wahl einer geeigneten Impulsdauer die Schicht 3, bestehend aus SiCₓH_{y}O_{z} aufgebaut wird.

Die Eigenschaft der Schicht hängt wesentlich von den Parametern "Pulsdauer" und "Konzentration des Reaktionsgases" ab. Generell werden bei kleinen Konzentrationen und langen Pulsdauem härtere Schichten abgeschieden, die eine wesentliche Steigerung der Inertheit bewirken. Bei hohen Konzentrationen und kleinen Pulsdauern werden weichere Schichten mit verbesserten Gleiteigenschaften abgeschieden.

Eine Prozeßsteuerung 13 steuert den Ablauf in bekannter Weise, bei dem zunächst aus der Zufuhranordnung 12 die Mischung aus Sauerstoff und dem Reaktionsgas in die Vakuumkammer 4 eingeleitet wird. Danach wird jeweils durch einen Mikrowellenimpuls 8a bis 8d ein Plasma in der Vakuumkammer 4 gezündet, welches die Moleküle des Reaktionsgases crackt. Die dabei entstehenden Crack-Produkte diffundieren zur nächst gelegenen Oberfläche, hier der Matte 1 mit dem Elastomerkompenten 2 und formen nach und nach die gewünschte Schicht 3. In der Impulspause bis zur Zündung des nächsten Impulses, die im Bereich von 10-100 ms liegt, werden jeweils die verbrauchten Reaktionsgase aus der Vakuumkammer nach Art eines Zweitaktmotors durch Absaugen mittels der Vakuumstufe 5 entfernt und durch frisches Reaktionsgas und Sauerstoff ersetzt.

Grundsätzlich kann auch eine Mehrfachschicht erzeugt werden. Dazu wird, sobald die genügende Schichtdicke für die erste Schicht erreicht ist, das zugehörige Reaktionsgas durch das zur Erzeugung der zweiten Schicht notwendige Reaktionsgas ersetzt. Zur Erzeugung eines unscharfen Überganges zwischen den beiden Schichten kann für einen gewissen Zeitraum ein Gemisch aus beiden Reaktionsgasen eingeleitet werden. Für gleichmäßige Übergänge kann man dabei den Anteil des ersten Reaktionsgases vermindern und gleichzeitig den Anteil des zweiten Reaktionsgases stetig bis auf den Nennwert erhöhen.

Die in Fig. 2 dargestellte PICVD-Technologie (Plasma-Impuls-Chemical-Vapor-Deposition) ist beispielsweise bekannt durch die DE 40 08 405 C1 und wird bislang insbesondere zur Erzeugung von Sperrschichten auf Kunststoffbehältern angewendet (DE 44 38 359 A1).

Die lineare Plasmaquelle nach Fig. 2 ist grundsätzlich durch die DE 38 30 249 C2 bekannt geworden, wobei die Steuerung der einzelnen Mikrowellenantennen 8a bis 8d gleichzeitig parallel oder durch das zeitversetzte Betreiben zweier benachbarter Mikrowellenantennen, z.B. gemäß der DE 40 34 211 erfolgt.

Nach dem Beschichten werden die Matten 1 aus der Vakuumkammer entfernt und einer weiteren Vorrichtung zugeführt, in der die Elastomerkomponenten aus der Matte herausgestanzt werden, was mit bekannten Techniken erfolgt.

Wenn erhöhte Sperreigenschaften gewünscht sind bzw. auch Transporteigenschaften verbessert werden sollen, kann die Rückseite der Matte 1 nach Fig. 1 - wie dort dargestellt - ebenfalls mit einer Sperrschicht 4 versehen werden, wobei sogar unterschiedliche Eigenschaften auf Vorder- und Rückseite durch die Wahl des Schichttyps eingestellt werden können. Entsprechende Anlagen, mit denen gleichzeitig zwei entsprechende Schichten aufgetragen werden können, sind bekannt.

## Patentansprüche

1. Verfahren zum Beschichten von Elastomerkomponenten für medizinische Zwecke, mit den Schritten:
- Herstellen einer ausgedehnt flächigen Matte aus dem Elastomerwerkstoff mit einer Vielzahl von zusammenhängend angeformten vorstehenden Elastomerkomponenten,
- Beschichten mindestens der Seite der Matte mit den vorstehenden Elastomerkomponenten mittels eines "kalten" Beschichtungsverfahrens, typischerweise dem plasmaunterstützten CVD-Verfahren (PECVD-Verfahren) in einem kontinuierlichen Prozeß mit einer Schicht, die eine Inertheit der Elastomerkomponenten und ihr Gleit- sowie Haftreibungsverhalten verbessert, und
- Ausstanzen der Elastomerkomponenten aus der Matte.

2. Verfahren nach Anspruch 1, **dadurch gekennzeichnet,** daß als Beschichtungsverfahren das Plasmaimpuls-CVD-Verfahren (PICVD-Verfahren) eingesetzt wird.

3. Verfahren nach Anspruch 1, **dadurch gekennzeichnet,** daß die Schicht eine Siliziumdioxid-Schicht ist, die auch Kohlenstoff, Wasserstoff und/oder Stickstoff enthält.

4. Verfahren nach Anspruch 2 und 3, **dadurch gekennzeichnet,** daß als Beschichtungsgas für das PICVD-Verfahren Tetramethyldisiloxan (TMDSO), Hexamethyldisiloxan (HMDSO) oder ähnliche Siloxane verwendet werden.

5. Verfahren nach Anspruch 2 und 3, **dadurch gekennzeichnet,** daß als Reaktionsgas für das PICVD-Verfahren Silazane verwendet werden.

6. Verfahren nach einem der Ansprüche 1 bis 5, **dadurch gekennzeichnet,** daß eine Matte mit angeformten Injektions- oder Infusions- oder Spritzen-Kolbenstopfen beschichtet wird.

7. Verfahren nach einem der Ansprüche 1 bis 6, dadurch gekennzeichnet, daß beide Seiten der Matte beschichtet werden.

8. Verfahren nach Anspruch 7, dadurch gekennzeichnet, daß die Schichttypen auf beiden Seiten der Matte unterschiedlich sind.

## Claims

1. Method of coating elastomeric components for medicinal purposes, with the steps of:
- producing an extended planar mat made of the elastomeric material with a plurality of abovementioned elastomeric components moulded together,
- coating at least that side of the mat with the abovementioned elastomeric components by means of a "cold" coating process, typically the plasma-assisted CVD process (PECVD process), in a continuous process, with a layer which improves the inertness of the elastomeric components and their sliding and static friction properties, and
- punching out the elastomeric components from the mat.

2. Process according to Claim 1, **characterized in that** the coating process used is the pulsed plasma CVD process (PICVD process).

3. Process according to Claim 1, **characterized in that** the layer is a silicon dioxide layer which also contains carbon, hydrogen and/or nitrogen.

4. Process according to Claim 2 or 3, **characterized in that** the coating gas used for the PICVD process is tetramethyldisiloxane (TMDSO), hexamethyldisiloxane (HMDSO) or similar siloxanes.

5. Process according to Claim 2 or 3, **characterized in that** the reaction gas used for the PICVD process is silazanes.

6. Process according to any of Claims 1 to 5, **characterized in that** the mat coated is one with moulded injection stoppers, or infusion stoppers or spray bottle stoppers.

7. Process according to any of Claims 1 to 6, characterized in that both sides of the mat are coated.

8. Process according to Claim 7, characterized in that the layer types on the two sides of the mat are different.

## Revendications

1. Procédé pour le revêtement de composants élastomères destinés à des fins médicales, comprenant les étapes :
- de fabrication d'une natte plane par allongement en substance élastomère présentant une multitude de composants élastomères en saillie moulés dessus en continu,
- de revêtement d'au moins le côté de la natte présentant les composants élastomères en saillie, au moyen d'un procédé de revêtement "à froid", de manière caractéristique le procédé CVD soutenu par plasma (le procédé PECVD) dans un procédé continu, d'une couche qui améliore le caractère inerte des composants élastomères et leur tenue au glissement ainsi que leur tenue au frottement statique et
- de découpage à la presse des composants élastomères de la natte.

2. Procédé selon la revendication 1, caractérisé en ce qu'on utilise comme procédé de revêtement le procédé CVD par impulsions de plasma (procédé PICVD).

3. Procédé selon la revendication 1, caractérisé en ce que la couche est une couche de dioxyde de silicium, qui contient également du carbone, de l'hydrogène et/ou de l'azote.

4. Procédé selon les revendications 2 et 3, caractérisé en ce qu'on utilise comme gaz de revêtement pour le procédé PICVD du tétraméthyldisiloxane (TMDSO), de l'hexaméthyldisiloxane (HMDSO) ou des siloxanes analogues.

5. Procédé selon les revendications 2 et 3, caractérisé en ce qu'on utilise comme gaz de réaction pour le procédé PICVD des silazanes.

6. Procédé selon l'une quelconque des revendications 1 à 5, caractérisé en ce qu'on revêt une natte présentant des bouchons pour pistons d'injection, ou de perfusion ou de seringue moulés dessus.

7. Procédé selon l'une quelconque des revendications 1 à 6, caractérisé en ce qu'on revêt les deux côtés de la natte.

8. Procédé selon la revendication 7, caractérisé en ce que les types de couches sur les deux côtés de la natte sont différents.
